# EUROPEAN PATENT SPECIFICATION

(11) **EP 2 740 438 B1**
(45) Date of publication and mention of the grant of the patent: **23.01.2019**
(21) Application number: 12196261.7
(22) Date of filing: 10.12.2012
(51) Int. Cl.: A61F 2/07

(54) **Stent graft having rougher portions and method of making the same**
Stentgraft mit raueren Abschnitten und Herstellungsverfahren dafür
Endoprothèse présentant des parties plus rugueuses et son procédé de fabrication

(43) Date of publication of application: 11.06.2014
(73) Proprietor: Abbott Laboratories Vascular Enterprises Limited, Terrace, Dublin 2 (IE)
(72) Inventor: Grandt, Axel, 72479 Straßberg (DE)
(74) Representative: Peters, Hajo

(56) References cited:
- WO-A2-01/82833
- US-B1- 6 254 632
- US-B1- 6 979 346

## Description

The invention relates to a stent graft having an expandable stent and a tissue graft, and a method of making the same.

### Background of the invention

In medicine, a natural conduit in a body may be locally flow constricted or damaged. A stent graft may be inserted into this natural conduit, in order to counteract such a flow constriction or to repair damage. A stent graft is an artificial tube-like device having a stent with meshed walls, the dimension of the stent, in particular the diameter, being expandable once it is positioned in the natural conduit appropriately. In the expanded shape, the stent holds the natural conduit open to allow the flow of body fluids or the access for surgery. The stent is covered with a tubular fabric or graft material which is supported by the stent. This way the graft material can close a damaged spot of the natural conduit.

When expanding the stent, the graft tissue is expanded in diameter. This expansion causes shrinkage of the graft tissue in length which is known as foreshortening. There already have been efforts to reduce this foreshortening effect. However, there is still potential for further improvement.

US6254632 describes an implantable medical device having protruding surface structures for drug delivery and cover attachment.

### Summary of the invention

It is an object of the present invention to provide a stent graft and a method for manufacturing the same, in order to reduce the foreshortening effect of graft material covering the stent.

This object is solved with a stent graft and a method for manufacturing the same according to the independent claims. Advantageous further developments are subject of the dependent claims.

According to an embodiment of the invention a stent graft is provided comprising a first expandable stent having a tubular wall, and a tissue graft disposed around the outside of the tubular wall, wherein the outside of the tubular wall is rougher at longitudinal end portions of the first stent compared to a center portion located at the outside of the tubular wall in between the longitudinal end portions. This provides the benefits that the graft material is held in an improved way to the end portions of the expandable stent while the stent is expanded, such that the shrinkage in length of the graft material is kept low or eliminated, while at the same time the graft material can slide at a center portion in between the end portions.

According to another embodiment of the invention, the stent graft further comprises a second expandable stent having a tubular wall, wherein the second expandable stent is concentrically arranged around the tissue graft. Due to the provision of a second stent arranged around the first stent and the graft material, the graft material is more securely held to the inner stent.

According to a yet further embodiment of the invention, the inside of the tubular wall of the second stent is rougher at longitudinal end portions of the second stent compared to a center portion located at the inside of the tubular wall in between the longitudinal end portions. This provides the same advantage for the second stent as mentioned above for the first stent.

According to a further embodiment of the invention, the first stent is constructed such that the respective tubular wall comprises at least three circumferential segments, wherein the end portions of the stent are formed by the two outermost circumferential segments.

According to a further embodiment of the invention, the second stent is constructed such that the respective tubular wall comprises at least three circumferential segments, wherein the end portions of the second stent are formed by the two outermost circumferential segments.

According to a further embodiment of the invention, the stent graft further comprises tie bars longitudinally interconnecting said plurality of circumferential segments.

According to a further embodiment of the invention, the rougher portions of the tubular wall are roughened by grinding.

According to a further embodiment of the invention, the rougher portions of the tubular wall are roughened by etching.

According to a further embodiment of the invention, the rougher portions of the tubular wall are roughened by milling.

According to a further embodiment of the invention, the center portion located in between the rougher longitudinal end portions is electropolished.

According to a further embodiment of the invention, the rougher portions of the tubular wall have a roughness of Rₐ > 0.5µm according to DIN EN ISO 4287.

According to a further embodiment of the invention, the rougher portions of the tubular wall have a roughness of R_{z} > 0.3µm according to DIN EN ISO 4287.

According to a further embodiment of the invention, a method for manufacturing a stent graft is provided, the method comprising the steps of providing a first expandable stent having a tubular wall, and disposing a tissue graft around the outside of the tubular wall, wherein the outside of the tubular wall is rougher at longitudinal end portions of the first stent compared to a center portion located at the outside of the tubular wall in between the longitudinal end portions. This method has the same benefits as already described in connection with the stent graft above.

According to a further embodiment of the invention, a method is provided comprising the step of providing a second expandable stent having a tubular wall, wherein the second expandable stent is concentrically arranged around the tissue graft, wherein the inside of the tubular wall of the second stent is rougher at longitudinal end portions of the second stent compared to a center portion located at the inside of the tubular wall in between the longitudinal end portions.

These and other embodiments are described in more detail with reference to the Figures.

### Brief description of the Figures

Fig. 1 schematically shows an expandable stent of a stent graft according to an exemplary embodiment of the invention;
Fig. 2 schematically shows a stent graft according to a first exemplary embodiment of the invention;
Fig. 3 schematically shows a stent graft according to a second exemplary embodiment of the invention, and
Fig. 4 shows an exemplary example of a rougher end portion of the stent of Fig. 1.

### Detailed description of exemplary embodiments of the invention

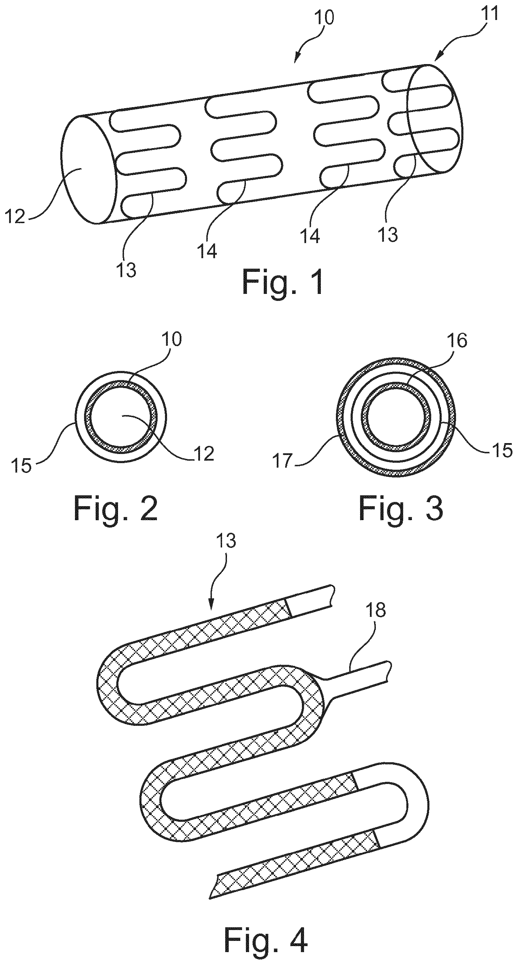

Fig. 1 schematically shows an expandable stent of a stent graft according to an exemplary embodiment of the invention. The expandable stent 10 has a tubular shape formed by a tubular wall 11 which forms a lumen 12 there through. The tubular wall 11 is formed by circumferential segments 13, 14. Each circumferential segment 13, 14 surrounds a longitudinal center axis of the tubular shaped stent as closed loop, wherein each segment 13, 14 includes a serpentine pattern, also known as a zigzag or Z pattern. The serpentines of the serpentine pattern extend within the cylindrical surface of the tubular shaped stent. The number of the segments 13, 14 is at least three, and can be any reasonable number above that. The stent 10 can be formed of metal. Alternative thereto, the stent 10 can also be constructed differently to this. Basically the invention can also be used beneficially in connection with other designs of expandable stents known from the state of the art. The expansion of the stent 10 can for example be realized by balloon expansion or by self-expansion of the stent 10 as both known from the state of the art.

According to a first exemplary embodiment of the invention (see Fig. 2) a stent graft can be provided comprising at least one expandable stent 10, such as the one shown in Fig. 1, and a tissue graft or membrane disposed concentrically around this stent 10. Alternatively, according to a second exemplary embodiment of the invention (see Fig. 3), a stent graft can be provided comprising an inner stent and an outer stent, both of which may be constructed as the stent 10 shown in Fig. 1, wherein a tissue graft or membrane is disposed concentrically in between the inner and the outer stent. The tissue graft or membrane can be biological tissue such as a mammary vein or other veins of an animal. Also a biological tissue other than a vein can be used for the tissue graft. Moreover, the tissue graft can be made of suitable synthetic polymers which are biocompatible to the human body.

Fig. 2 schematically shows a stent graft according to a first exemplary embodiment of the invention. The stent graft is formed by arranging a tissue graft 15 concentrically around the stent 10 shown and described in connection with Fig. 1. The outside of the tubular wall 11 (the side of the tubular wall facing radially outwards) is in direct contact with the tissue graft 15. According to the invention, end portions of the outside of the tubular wall 11 are rougher than a center portion, as will be described in more detail in connection with Fig. 4. Please note that Fig. 2 is illustrating the stent graft with a space in between the stent 10 and the surrounding tissue graft 15, however, Fig. 2 is only a schematic drawing and according to the invention, the outside of stent 10 is in direct contact with the tissue graft 15 as mentioned above.

Fig. 3 schematically shows a stent graft according to a second exemplary embodiment of the invention. An inner stent 16 which is constructed like the stent 10 described in Fig. 1 is provided concentrically inside an outer stent 17 which is also constructed like the stent 10 described in Fig. 1. In between the inner stent 16 and the outer stent 17, a tissue graft 15 is arranged, wherein the inner side of the tissue graft 15 (the side facing radially inwards), is in direct contact to the outside of the tubular wall of the inner stent 16, and wherein the outer side of the tissue graft 15 (the side facing radially outwards), is in direct contact to the inside (the side facing radially inwards) of the tubular wall of the outer stent 17. According to the invention, end portions of the outside of the tubular wall of the inner stent 16 and end portions of the inside of the tubular wall of the outer stent 17 are rougher than respective center portions of these surfaces, as will be described in more detail in connection with Fig. 4. Please note that Fig. 3 is illustrating the stent graft with a space in between the inner stent 16 and the surrounding tissue graft 15, and in between the outer stent 17 and the tissue graft 15, however, Fig. 3 is only a schematic drawing and according to the invention, the outside of inner stent 16 as well as the inside of the outer stent 17 is in direct contact with the tissue graft 15 as mentioned above.

In order to reduce the foreshortening of the material of the tissue graft 15 in expansion of the covered stents 10, 16, the present invention proposes to provide the end portions of the tubular wall 11 on the surfaces which are in direct contact with the tissue graft 15 with a higher roughness than a center part of these contact surfaces. The end portions can for example be formed by part of the outermost segments 13 or the entire outermost segments 13 of the stent 10 shown in Fig. 1, and the center part can be formed by the segments 14 arranged in between the outermost segment 13. Or in other words, end portions of the outside of the tubular wall 11 of the stent 10 or the inner stent 16, or end portions of the inside of the tubular wall 11 of the outer stent 17, are rougher than the respective center portions on these respective surfaces in between the end portions.

Fig. 4 shows an exemplary example of a rougher end portion. In this Figure, the left outer segment 13 of Fig. 1 is shown in a roughened state. In order to achieve the rougher end portions, there are basically two approaches. One is to keep the end portions unmachined and to electropolish the center portion. The other approach is to optionally electropolish the entire surface of the tubular wall 11 of the stent 10, 16, 17, which surface is in contact with the tissue graft 15 and to roughen the mentioned end portions thereafter by means of at least one of sandblasting, grinding, etching, laser ablation, or milling. The circumferential segments 13, 14 may be connected by longitudinal tie bars 18 which connect two adjacent segments 13, 14.

In the foregoing it was described that the rougher end portions may for example be part of (such as the outer half of the outermost segments) or the entire inner/outer surface of the outermost circumferential segments 13. However, the end portions can also be formed by more than one outer segment 13 per stent end. Part of the outermost stent is to be understood as part of the outermost stent such that a rougher area extends as a closed loop around the entire circumference limited on one side by the end of the stent and extending on the opposite side a certain extent in the longitudinal direction (with respect to the center axis of the stent) on the outermost segment.

According to another example, which is not claimed yet, the surface of the tubular wall 11 of stent 10, 16, 17 which is in contact with the graft tissue 15 may be roughened entirely, instead of only their end portions.

While the invention has been illustrated and described in detail in the drawings and foregoing description, such illustration and description are to be considered illustrative or exemplary and not restrictive and it is not intended to limit the invention to the disclosed embodiments. The mere fact that certain measures are recited in mutually different dependent claims does not indicate that a combination of these measures cannot be used advantageously.

## Claims

1. Stent graft comprising
a first expandable stent (10; 16) having a tubular wall (11), and
a tissue graft (15) disposed around the outside of the tubular wall (11), wherein the outside of the tubular wall (11) is rougher at longitudinal end portions of the first stent (10; 16) compared to a center portion located in between the longitudinal end portions,
wherein the rougher portions of the tubular wall (11) are roughened by at least one of grinding, etching, and milling.

2. Stent graft according to claim 1, further comprising
a second expandable stent (17) having a tubular wall (11), wherein the second expandable stent (17) is concentrically arranged around the tissue graft (15).

3. Stent graft according to claim 2, wherein the inside of the tubular wall (11) of the second stent (17) is rougher at longitudinal end portions of the second stent (17) compared to a center portion located in between the longitudinal end portions.

4. Stent graft according to one of the preceding claims, wherein the first stent (16) is constructed such that the respective tubular wall (11) comprises at least three circumferential segments (13, 14), wherein the end portions of the stent (10; 16) are formed by the two outermost circumferential segments (13).

5. Stent graft according to claim 3, wherein the second stent (17) is constructed such that the respective tubular wall (11) comprises at least three circumferential segments (13, 14), wherein the end portions of the second stent (17) are formed by the two outermost circumferential segments (13).

6. Stent graft according to at least one of claims 4 or 5, further comprising tie bars (18) longitudinally interconnecting said plurality of circumferential segments (13, 14).

7. Stent graft according to one of the proceeding claims, wherein the center portion located in between the rougher longitudinal end portions is electropolished.

8. Stent graft according to one of the proceeding claims, wherein the rougher portions of the tubular wall (11) have a roughness of Rₐ > 0.5µm according to DIN EN ISO 4287.

9. Stent graft according to one of the proceeding claims, wherein the rougher portions of the tubular wall (11) have a roughness of R_{z} > 0.3µm according to DIN EN ISO 4287.

10. Method for manufacturing a stent graft comprising the steps of
providing a first expandable stent (10; 16) having a tubular wall (11), and
disposing a tissue graft (15) around the outside of the tubular wall (11), wherein the outside of the tubular wall (11) is rougher at longitudinal end portions of the first stent (16) compared to a center portion located in between the longitudinal end portions, wherein the rougher portions of the tubular wall (11) are roughened by at least one of grinding, etching, and milling.

11. Method according to claim 10, further comprising the step of
providing a second expandable stent (17) having a tubular wall (11), wherein the second expandable stent (17) is concentrically arranged around the tissue graft (15), wherein the inside of the tubular wall (11) of the second stent (17) is rougher at longitudinal end portions of the second stent (17) compared to a center portion located in between the longitudinal end portions.

12. Method according to claim 10 or 11, wherein the center portion located in between the rougher longitudinal end portions is electropolished.

13. Method according to one of claims 10 to 12, wherein the rougher portions of the tubular wall have a roughness of Rₐ > 0.5µm and/or R_{z} > 0.3µm according to DIN EN ISO 4287.

## Patentansprüche

1. Stent-Prothese (stent graft), umfassend
einen ersten expandierbaren Stent (10; 16) mit einer rohrförmigen Wand (11), und
eine Gewebe-Prothese (15), die um die Außenseite der rohrförmigen Wand (11) angebracht ist, wobei die Außenseite der rohrförmigen Wand (11) an Längsendabschnitten des ersten Stents (10; 16) im Vergleich zu einem Mittelabschnitt, der zwischen den Längsendabschnitten lokalisiert ist, rauer ist, wobei die raueren Abschnitte der rohrförmigen Wand (11) durch mindestens eines von Schleifen, Ätzen und Fräsen aufgeraut sind.

2. Stent-Prothese (stent graft) nach Anspruch 1, ferner umfassend einen zweiten expandierbaren Stent (17) mit einer rohrförmigen Wand (11), wobei der zweite expandierbare Stent (17) konzentrisch um die Gewebe-Prothese (15) angeordnet ist.

3. Stent-Prothese (stent graft) nach Anspruch 2, wobei die Innenseite der rohrförmigen Wand (11) des zweiten Stents (17) an Längsendabschnitten des zweiten Stents (17) im Vergleich zu einem Mittelabschnitt, der zwischen den Längsendabschnitten lokalisiert ist, rauer ist.

4. Stent-Prothese (stent graft) nach einem der vorstehenden Ansprüche, wobei der erste Stent (16) so aufgebaut ist, dass die jeweilige rohrförmige Wand (11) mindestens drei Umfangssegmente (13, 14) umfasst, wobei die Endabschnitte des Stents (10; 16) durch die zwei äußersten Umfangssegmente (13) gebildet sind.

5. Stent-Prothese (stent graft) nach Anspruch 3, wobei der zweite Stent (17) so aufgebaut ist, dass die jeweilige rohrförmige Wand (11) mindestens drei Umfangssegmente (13, 14) umfasst, wobei die Endabschnitte des zweiten Stents (17) durch die zwei äußersten Umfangssegmente (13) gebildet sind.

6. Stent-Prothese (stent graft) nach mindestens einem der Ansprüche 4 oder 5, das ferner Zugstangen (18) umfasst, die die Vielzahl von Umfangssegmenten (13, 14) in Längsrichtung miteinander verbinden.

7. Stent-Prothese (stent graft) nach einem der vorstehenden Ansprüche, wobei der Mittelabschnitt, der zwischen den raueren Längsendabschnitten lokalisiert ist, elektropoliert ist.

8. Stent-Prothese (stent graft) nach einem der vorstehenden Ansprüche, wobei die raueren Abschnitte der rohrförmigen Wand (11) eine Rauheit von Rₐ > 0,5 µm gemäß DIN EN ISO 4287 aufweisen.

9. Stent-Prothese (stent graft) nach einem der vorstehenden Ansprüche, wobei die raueren Abschnitte der rohrförmigen Wand (11) eine Rauheit von R_{z} > 0,3 µm gemäß DIN EN ISO 4287 aufweisen.

10. Verfahren zur Herstellung einer Stent-Prothese (stent grafts), das die folgenden Schritte umfasst:
Bereitstellen eines ersten expandierbaren Stents (10; 16) mit einer rohrförmigen Wand (11), und
Anbringen einer Gewebe-Prothese (15) um die Außenseite der rohrförmigen Wand (11), wobei die Außenseite der rohrförmigen Wand (11) an Längsendabschnitten des ersten Stents (16) im Vergleich zu einem Mittelabschnitt, der zwischen den Längsendabschnitten lokalisiert ist, rauer ist, wobei die raueren Abschnitte der rohrförmigen Wand (11) durch mindestens eines von Schleifen, Ätzen und Fräsen aufgeraut sind.

11. Verfahren nach Anspruch 10, das ferner den folgenden Schritt umfasst:
Bereitstellen eines zweiten expandierbaren Stents (17) mit einer rohrförmigen Wand (11), wobei der zweite expandierbare Stent (17) konzentrisch um die Gewebe-Prothese (15) angeordnet ist, wobei die Innenseite der rohrförmigen Wand (11) des zweiten Stents (17) an Längsendabschnitten des zweiten Stents (17) im Vergleich zu einem Mittelabschnitt, der zwischen den Längsendabschnitten lokalisiert ist, rauer ist.

12. Verfahren nach Anspruch 10 oder 11, wobei der Mittelabschnitt, der zwischen den raueren Längsendabschnitten lokalisiert ist, elektropoliert ist.

13. Verfahren nach einem der Ansprüche 10 bis 12, wobei die raueren Abschnitte der rohrförmigen Wand eine Rauheit von Rₐ > 0,5 µm und/oder R_{z} > 0,3 µm gemäß DIN EN ISO 4287 aufweisen.

## Revendications

1. Endoprothèse couverte comprenant
une première endoprothèse expansible (10 ; 16) présentant une paroi tubulaire (11) et
une greffe tissulaire (15) disposée autour de l'extérieur de la paroi tubulaire (11), l'extérieur de la paroi tubulaire (11) étant plus rugueux au niveau de parties d'extrémité longitudinale de la première endoprothèse (10 ; 16) par rapport à une partie centrale située entre les parties d'extrémité longitudinale, les parties plus rugueuses de la paroi tubulaire (11) étant rendues rugueuses par au moins l'un parmi un ponçage, une gravure et un fraisage.

2. Endoprothèse couverte selon la revendication 1, comprenant en outre une deuxième endoprothèse expansible (17) présentant une paroi tubulaire (11), la deuxième endoprothèse expansible (17) étant agencée de manière concentrique autour de la greffe tissulaire (15).

3. Endoprothèse couverte selon la revendication 2, l'intérieur de la paroi tubulaire (11) de la deuxième endoprothèse (17) étant plus rugueux au niveau de parties d'extrémité longitudinale de la deuxième endoprothèse (17) par rapport à une partie centrale située entre les parties d'extrémité longitudinale.

4. Endoprothèse couverte selon l'une quelconque des revendications précédentes, la première endoprothèse (16) étant construite de telle sorte que la paroi tubulaire respective (11) comprend au moins trois segments circonférentiels (13, 14), les parties d'extrémité de l'endoprothèse (10; 16) étant formées par les deux segments circonférentiels les plus extérieurs (13).

5. Endoprothèse couverte selon la revendication 3, la deuxième endoprothèse (17) étant construite de telle sorte que la paroi tubulaire respective (11) comprend au moins trois segments circonférentiels (13, 14), les parties d'extrémité de la deuxième endoprothèse (17) étant formées par les deux segments circonférentiels les plus extérieurs (13).

6. Endoprothèse couverte selon au moins l'une quelconque des revendications 4 ou 5, comprenant en outre des barres de liaison (18) interconnectant longitudinalement ladite pluralité de segments circonférentiels (13, 14).

7. Endoprothèse couverte selon l'une quelconque des revendications précédentes, la partie centrale située entre les parties d'extrémité longitudinale plus rugueuses étant polie électrolytiquement.

8. Endoprothèse couverte selon l'une quelconque des revendications précédentes, les parties plus rugueuses de la paroi tubulaire (11) présentant une rugosité de Rₐ > 0,5 µm selon la norme DIN EN ISO 4287.

9. Endoprothèse couverte selon l'une quelconque des revendications précédentes, les parties plus rugueuses de la paroi tubulaire (11) présentant une rugosité de R_{z} > 0,3 µm selon la norme DIN EN ISO 4287.

10. Procédé de fabrication d'une endoprothèse couverte comprenant les étapes de
fourniture d'une première endoprothèse expansible (10 ; 16) présentant une paroi tubulaire (11) et
mise en place d'une greffe tissulaire (15) autour de l'extérieur de la paroi tubulaire (11), l'extérieur de la paroi tubulaire (11) étant plus rugueux au niveau de parties d'extrémité longitudinale de la première endoprothèse (16) par rapport à une partie centrale située entre les parties d'extrémité longitudinale, les parties plus rugueuses de la paroi tubulaire (11) étant rendues rugueuses par au moins l'un parmi un ponçage, une gravure et un fraisage.

11. Procédé selon la revendication 10, comprenant en outre l'étape de fourniture d'une deuxième endoprothèse expansible (17) présentant une paroi tubulaire (11), la deuxième endoprothèse expansible (17) étant agencée de manière concentrique autour de la greffe tissulaire (15), l'intérieur de la paroi tubulaire (11) de la deuxième endoprothèse (17) étant plus rugueux au niveau de parties d'extrémité longitudinale de la deuxième endoprothèse (17) par rapport à une partie centrale située entre les parties d'extrémité longitudinale.

12. Procédé selon la revendication 10 ou 11, la partie centrale située entre les parties d'extrémité longitudinale plus rugueuses étant polie électrolytiquement.

13. Procédé selon l'une quelconque des revendications 10 à 12, les parties plus rugueuses de la paroi tubulaire présentant une rugosité de Rₐ > 0,5 µm et/ou R_{z} > 0,3 µm selon la norme DIN EN ISO 4287.
